# EUROPEAN PATENT APPLICATION

(11) **EP 2 422 686 A1**
(43) Date of publication of application: **29.02.2012**
(21) Application number: 11179072.1
(22) Date of filing: 26.08.2011
(51) Int. Cl.: A61B 1/015, A61B 1/12, A61B 1/00

(54) **Endoscope, mantle tube, and endoscope system**

(30) Priority: 30.08.2010 JP 2010192748
(71) Applicant: Fujifilm Corporation, Minato-ku Tokyo 106-8620 (JP)
(72) Inventor: Torisawa, Nobuyuki, Ashigarakami-gun, Kanagawa 258-8538 (JP)
(74) Representative: Klunker . Schmitt-Nilson . Hirsch

(57) **Abstract**

Provided is an endoscope system including: a mantle tube into which an insertion part of an endoscope is inserted; a fluid flow channel which is formed of a space between an outer surface of the endoscope inserted into the mantle tube and an inner surface of the mantle tube; a sealing member which seals the fluid flow channel; and a pipe line which is formed inside of the insertion part so as to communicate between the fluid flow channel and a nozzle formed on a leading end surface of the insertion part, and supply a fluid from the fluid flow channel to the nozzle, and further including a first fixing device which fixes the insertion part at a position at which a leading end of the insertion part is not exposed to an outside from a leading end of the mantle tube. Accordingly, it is possible to efficiently supply a cleaning liquid without providing a special flow channel when the cleaning liquid is supplied through the mantle tube, and facilitate the insertion of the insertion part of the endoscope into the mantle tube.

## Description

### BACKGROUND OF THE INVENTION

### Field of the Invention

The present invention relates to an endoscope, a mantle tube, and an endoscope system, and more particularly, to an endoscope including a mantle tube such as an oversheath, an overtube, or a trocar, into which an insertion part of the endoscope is inserted, the mantle tube, and an endoscope system.

### Description of the Related Art

In general, an endoscope broadly includes: a main body operation part to be gripped for operation by an operator; an insertion part to be inserted into a body cavity and the like, the main body operation part and the insertion part being connected to each other; and a universal cord for connecting with a connector part and the like which is pulled out from the main body operation part. The universal cord is extended from the main body operation part, and another end part thereof is detachably connected to a light source apparatus (a light source apparatus and a processor).

A leading end part of the insertion part to be inserted into an abdominal cavity includes: an illumination device which illuminates the inside of the body cavity which is a dark place; and an observation device which observes the inside of the body cavity under the illumination of the illumination device. The illumination device includes: a lens for illumination which is attached to an illumination window provided on a leading end surface of the leading end part; and a light guide having a light emission end which faces the lens for illumination. The observation device includes: an objective optical system which faces an observation window placed in the vicinity of the illumination window; and a solid state image pick-up element (CCD) which is placed at an imaging position of the objective optical system.

In an endoscope system including such an endoscope as described above, a pipe-like mantle tube such as a trocar or a sheath is used for facilitating the insertion of the endoscope into the body cavity, the trocar guiding an endoscope for observation to the inside of the body cavity, the sheath being inserted into the abdominal cavity by means of the guide of the trocar with an insertion part of a rigid endoscope being inserted thereinto.

Incidentally, a body fluid and the like exist inside of the body cavity into which the insertion part is inserted, and if the body fluid or other disfiguring substance attaches to the observation window, the observation field of view is blocked. Therefore, normally, the endoscope is provided with a cleaning mechanism of the observation window. At the time of cleaning of the observation window, first, a cleaning liquid, normally cleaning water, is supplied to the observation window to thereby wash away the disfiguring substance, and then, pressurized air is jetted toward the observation window in order to remove water drops attaching to the observation window. Therefore, in the endoscope, the leading end part of the insertion part is provided with a pipe line for supplying the cleaning liquid, the pressurized air, and the like.

For example, Japanese Patent Application Laid-Open No. 2009-189496 describes a rigid endoscope including a cleaning apparatus which includes a water supply sheath into which an insertion part is inserted, and cleans, with cleaning water, a transparent illumination window and a transparent observation window placed on a leading end surface of the insertion part. In this rigid endoscope, a pipe-like connection part is placed in a base end part of the water supply sheath, and a water supply tube is connected to a side part of the connection part. The water supply tube is connected to an external water supply tank, and gas is supplied to the water supply tank via a gas supply tube, whereby the cleaning water stored in the water supply tank is supplied to the connection part via the water supply tube by means of a pressure of the gas. The cleaning water supplied to the connection part is guided to the leading end side of the insertion part by a water supply channel which is formed in a thick portion of a tube main body of the water supply sheath, whereby the illumination window and the observation window are cleaned with the cleaning water.

In addition, Japanese Patent Application Laid-Open No. 2008-220775 describes that a lubricant such as water is injected from an injection port provided on the base end side of an insertion support tool through which an insertion part of an endoscope is inserted, to thereby reduce friction between an inner circumferential surface of the insertion support tool and an outer circumferential surface of the insertion part, so that the insertion of the insertion part is facilitated.

However, according to Japanese Patent Application Laid-Open No. 2009-189496, the water supply channel is formed in the thick portion of the water supply sheath which is a mantle tube, and the observation window and the like cannot be cleaned sufficiently with the cleaning water supplied from such a narrow water supply channel. In addition, in order to form the water supply channel, a wall of the water supply sheath needs to be made thicker, which leads to a problem that a diameter of the endoscope to be inserted into the water supply sheath needs to be made smaller accordingly.

In addition, according to Japanese Patent Application Laid-Open No. 2008-220775, although the endoscope inserted into the insertion support tool which is a mantle tube can be smoothly moved within the insertion support tool, there is a problem that, particularly, the insertion of a bending part formed of a flexible member at the leading end is difficult when the endoscope is inserted into the insertion support tool (mantle tube).

The present invention has been made in view of the above-mentioned circumstances, and therefore has an object to provide an endoscope, a mantle tube, and an endoscope system which are capable of: efficiently supplying a cleaning liquid without providing a special flow channel when the cleaning liquid for cleaning a surface of a leading end part of the endoscope is supplied through the mantle tube into which the endoscope is inserted; and facilitating the insertion of an insertion part of the endoscope into the mantle tube.

### SUMMARY OF THE INVENTION

In order to achieve the above-mentioned object, a first aspect of the present invention provides an endoscope system including: a mantle tube into which an insertion part of an endoscope is inserted; a fluid flow channel which is formed of a space between an outer surface of the endoscope inserted into the mantle tube and an inner surface of the mantle tube; a sealing member which seals the fluid flow channel; and a pipe line which is formed inside of the insertion part so as to communicate between the fluid flow channel and a nozzle formed on a leading end surface of the insertion part, and supply a fluid from the fluid flow channel to the nozzle.

With this feature, when a cleaning liquid for cleaning the surface of the leading end part of the endoscope is supplied through the mantle tube into which the endoscope is inserted, it is possible to efficiently supply the cleaning liquid without providing a special flow channel.

In addition, according to a second aspect of the present invention, the sealing member is provided on the inner surface of the mantle tube, and seals a portion between a side surface of the insertion part and the inner surface of the mantle tube.

As described above, because the sealing member is provided to the mantle tube, in the case where the endoscope is used alone without using the mantle tube, it is possible to eliminate a resistance which is caused by the sealing member when the endoscope is inserted into a body cavity.

In addition, according to a third aspect of the present invention, the sealing member is provided on a side surface of the insertion part, and seals a portion between the side surface of the insertion part and the inner surface of the mantle tube.

As described above, because the sealing member is provided to the insertion part, it is possible to reliably secure the fluid flow channel irrespective of a relative position between the insertion part and the mantle tube.

In addition, according to a fourth aspect of the present invention, the sealing member is an O-ring which is placed on the inner surface of the mantle tube on a leading end side from a position at which the pipe line formed inside of the insertion part communicates with the fluid flow channel.

With this feature, water-tightness of the fluid flow channel is secured.

In addition, according to a fifth aspect of the present invention, the fluid flow channel is configured by a spiral groove which is formed on the inner surface of the mantle tube continuously from a base end side to a leading end side.

In addition, according to a sixth aspect of the present invention, the fluid flow channel is configured by a linear groove which is formed on the inner surface of the mantle tube continuously in a longitudinal direction from a base end side to a leading end side.

With this feature, it is possible to reduce a fluid remaining inside of the fluid flow channel, to thereby prevent a sudden trouble caused by the remaining fluid.

In addition, according to a seventh aspect of the present invention, the mantle tube is one of: a sheath into which the insertion part is inserted; and a trocar which is a support member for inserting the insertion part into a body to be examined.

In addition, according to an eighth aspect of the present invention, the endoscope system further includes a first fixing device which fixes the insertion part at a position at which a leading end of the insertion part is not exposed to an outside from a leading end of the mantle tube.

With this feature, for example, it is possible to easily insert the insertion part into the trocar in the state where the insertion part is inserted into the sheath serving as the mantle tube so that the leading end of the insertion part is not exposed to the outside from the leading end of the sheath.

In addition, according to a ninth aspect of the present invention, the first fixing device includes: an O-ring which is placed at a predetermined position on the inner surface of the mantle tube; and a groove which is formed at a predetermined position of the insertion part so as to engage with the O-ring.

With this feature, for example, it is possible to perform, with a simple configuration, temporary fixing when the insertion part which is inserted into the sheath is inserted into the trocar.

In addition, according to a tenth aspect of the present invention, the endoscope system further includes a second fixing device which fixes the insertion part at a position at which the leading end of the insertion part is exposed to the outside from the leading end of the mantle tube.

With this feature, for example, the insertion part which is inserted into the sheath is inserted into the trocar, and then the insertion part is further inserted into the body cavity, whereby observation and the like can be performed.

In addition, according to an eleventh aspect of the present invention, the second fixing device includes: an O-ring which is placed at a predetermined position on the inner surface of the mantle tube; and a groove which is formed at a predetermined position of the insertion part so as to engage with the O-ring.

With this feature, for example, it is possible to perform, with a simple configuration, actual fixing of the insertion part when the observation and the like are actually performed.

In addition, according to a twelfth aspect of the present invention, the mantle tube is a sheath into which the insertion part is inserted.

In addition, similarly, in order to achieve the above-mentioned object, a thirteenth aspect of the present invention provides an endoscope including an insertion part, the insertion part including a side surface on which a sealing member which seals a fluid flow channel is provided, the fluid flow channel being formed of a space between an outer surface of the endoscope and an inner surface of a mantle tube by inserting the insertion part of the endoscope into the mantle tube.

In addition, similarly, in order to achieve the above-mentioned object, a fourteenth aspect of the present invention provides a mantle tube into which an insertion part of an endoscope is inserted, the mantle tube including an inner surface on which a sealing member which seals a fluid flow channel is provided, the fluid flow channel being formed of a space between an outer surface of the endoscope inserted into the mantle tube and the inner surface of the mantle tube.

With this feature, when the cleaning liquid for cleaning the surface of the leading end part of the endoscope is supplied through the mantle tube into which the endoscope is inserted, it is possible to efficiently supply the cleaning liquid without providing a special flow channel.

As described above, according to the present invention, it is possible to efficiently supply the cleaning liquid without providing a special flow channel when the cleaning liquid for cleaning the surface of the leading end part of the endoscope is supplied through the mantle tube into which the endoscope is inserted. Moreover, in the case where the endoscope system includes the first fixing device which fixes the insertion part at a position at which the leading end of the insertion part is not exposed to the outside from the leading end of the mantle tube, for example, it is possible to easily insert the insertion part into the trocar in the state where the insertion part is inserted into the sheath serving as the mantle tube so that the leading end of the insertion part is not exposed to the outside from the leading end of the sheath.

### BRIEF DESCRIPTION OF THE DRAWINGS

Fig. 1 is an overall configuration view illustrating an embodiment of an endoscope system including a mantle tube according to the present invention;
Fig. 2 is a perspective view illustrating, in an enlarged manner, the vicinity of a hand-side operation part of a rigid endoscope with an insertion part being inserted into a sheath;
Fig. 3 is a side view including a partial cross sectional view, which corresponds to Fig. 2;
Fig. 4 is a plan view illustrating a leading end surface of a leading end hard part of the insertion part;
Fig. 5 is a configuration view illustrating a supply system which supplies a cleaning liquid and CO₂ via a fluid pipe line;
Fig. 6 is a cross sectional view illustrating a leading end part of the sheath into which the insertion part of the endoscope is inserted;
Fig. 7 is a cross sectional view illustrating a state where the leading end of the insertion part is inserted so as not to protrude from a leading end opening of the sheath and then is temporarily fixed;
Fig. 8 is a cross sectional view illustrating a state where the insertion part is actually fixed to the sheath;
Fig. 9 is a cross sectional view illustrating a state where the insertion part is inserted into a trocar;
Fig. 10 is a cross sectional view which is perpendicular to the longitudinal direction with the insertion part being inserted into the trocar;
Fig. 11 is a perspective view in which part of an inner wall of the trocar is opened; and
Figs. 12A and 12B each illustrate a state where a single groove is formed in the longitudinal direction on the inner wall of the trocar, Fig. 12A is a cross sectional view in the longitudinal direction, and Fig. 12B is a cross sectional view taken along a broken line 12B-12B in Fig. 12A.

### DETAILED DESCRIPTION OF THE PREFERRED EMBODIMENTS

Hereinafter, an endoscope, a mantle tube, and an endoscope system according to the present invention are described in detail with reference to the attached drawings.

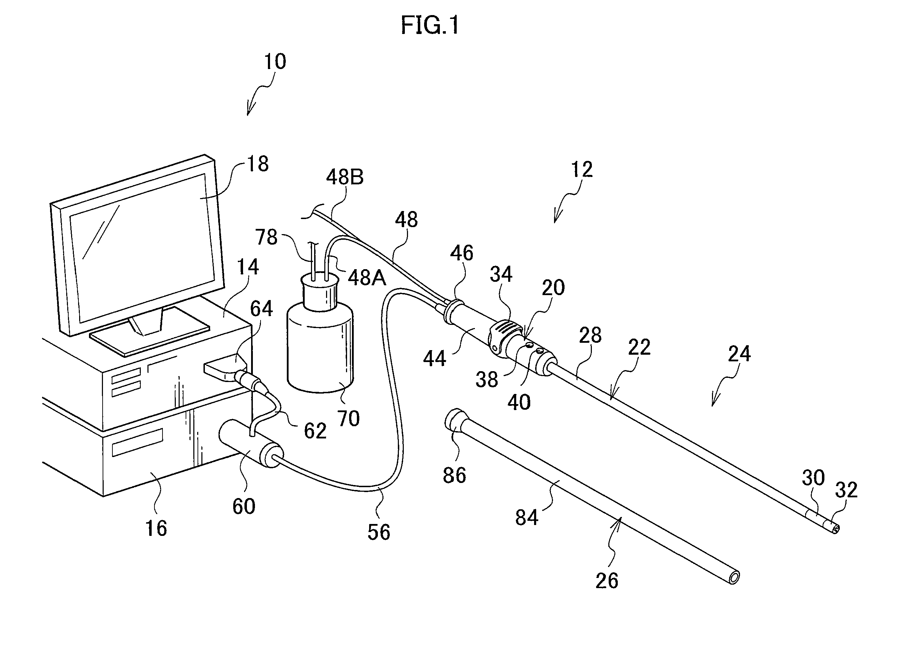

Fig. 1 is an overall configuration view illustrating an embodiment of the endoscope system including the endoscope and the mantle tube according to the present invention.

As illustrated in Fig. 1, an endoscope system 10 of the present embodiment includes an endoscope 12, a processor apparatus 14, a light source apparatus 16, and a monitor 18. It should be noted that the endoscope is assumed to be a flexible endoscope here, but may be a rigid endoscope.

The endoscope 12 includes: an endoscope main body 24 including a hand-side operation part (operation part) 20 to be gripped by an operator and an insertion part 22 to be inserted into an abdominal cavity; and a sheath (oversheath) 26 which is a mantle tube of the endoscope into which the insertion part 22 is inserted.

At the time of an operation, the insertion part 22 is inserted into the sheath 26, and the sheath 26 into which the insertion part 22 is inserted is inserted into a trocar (not shown) which is caused in advance to penetrate through the abdominal cavity of a patient. As a result, the leading end of the insertion part 22 is inserted into the abdominal cavity of the patient. It should be noted that the present invention provides a technique for facilitating the insertion into the trocar, of the sheath 26 into which the insertion part 22 is inserted, and this technique will be described later.

The insertion part 22 is connected to a leading end part of the hand-side operation part 20. Then, the insertion part 22 includes respective parts of a rigid part 28, a bending part 30, and a leading end hard part 32 in the stated order from its base end (on the hand-side operation part 20 side) to its leading end (on the side to be inserted into the abdominal cavity). As publicly known, the bending part 30 is configured by coupling a plurality of bending pieces which are each formed into a ring-like shape, and an angle knob (operation member) 34 which is rotatably attached to the hand-side operation part 20 is rotated, to thereby push and pull a wire insertedly provided inside of the insertion part 22 and thus bend the wire in a vertical direction or a horizontal direction. As a result, the leading end hard part 32 can be caused to face a desired direction inside of the abdominal cavity.

In addition, a water supply button 38 and a gas supply button 40 are attached to the hand-side operation part 20. A base end pipe part 44 of the hand-side operation part 20 functions as a grip part to be actually gripped by the operator, and a flange-like grip end 46 is formed at an end of the grip part.

In addition, a fluid pipe line 48 and a universal cord 56 are connected to the grip end 46 of the hand-side operation part 20. The fluid pipe line 48 serves to supply a cleaning liquid and CO₂ gas to the insertion part 22 side, and are branched into two pipe lines 48A and 48B. One pipe line 48A is attached to a water supply tank 70, and another pipe line 48B is connected to a gas pipe (not shown in Fig. 1). In addition, a pipe line 78 which supplies the CO₂ gas to the water supply tank 70 is connected to the water supply tank 70. A supply system which supplies the cleaning liquid and the CO₂ gas will be described in detail later.

In addition, an LG connector 60 is connected to an end of the universal cord 56. A signal cable 62 is connected so as to extend from a side part of the LG connector 60, and an electric connector 64 is connected to an end of the signal cable 62. The LG connector 60 is connected to a connector (not shown) of the light source apparatus 16, and the electric connector 64 is connected to a connector (not shown) of the processor apparatus 14. In addition, the processor apparatus 14 is electrically connected to the light source apparatus 16 and the monitor 18, subjects image data taken by the endoscope main body 24 to image processing, and displays the resultant image on the monitor 18.

In addition, the sheath 26 includes a pipe 84 and a connection part 86 connected to a base end part of the pipe 84. In the case of using the endoscope system 10, the insertion part 22 of the endoscope 12 is inserted into the pipe 84, and the connection part 86 of the sheath 26 is detachably connected to the hand-side operation part 20 of the endoscope 12.

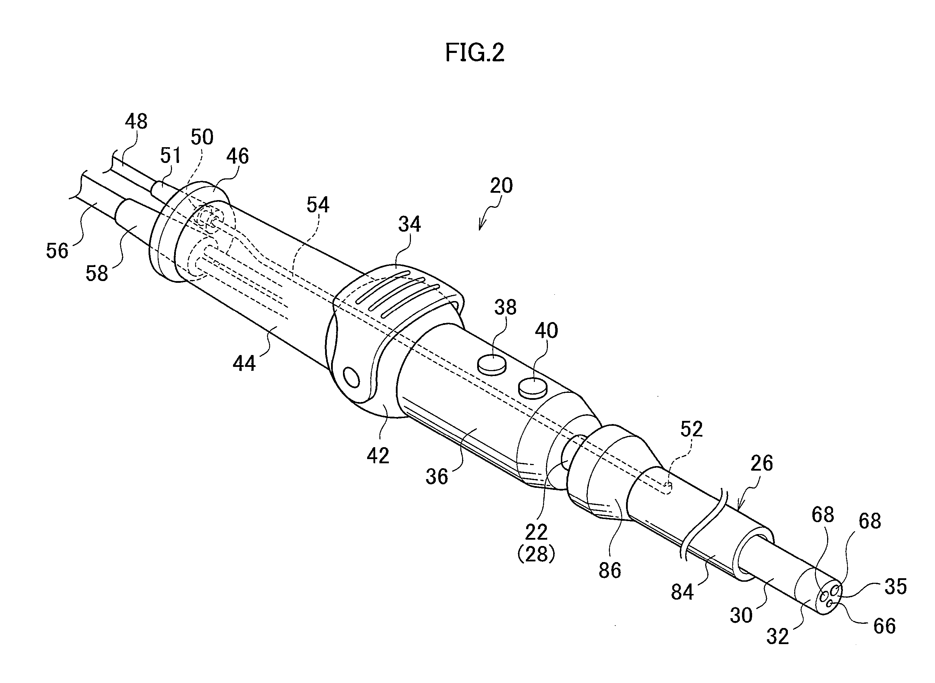

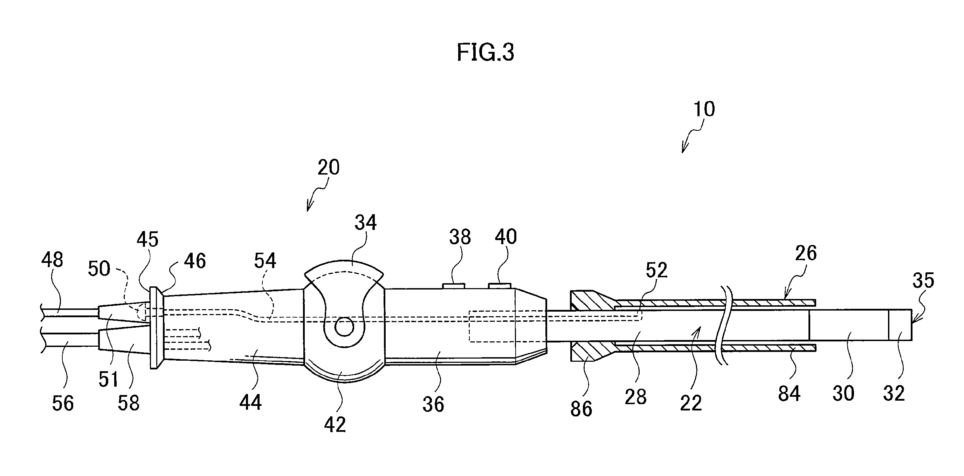

Fig. 2 is a perspective view illustrating, in an enlarged manner, the vicinity of the hand-side operation part 20 of the endoscope 12 with the insertion part 22 being inserted into the sheath 26. Fig. 3 is a side view including a partial cross sectional view, which corresponds to Fig. 2. It should be noted that, in Fig. 2 and Fig. 3, for ease of understanding, the connection part 86 of the sheath 26 is illustrated so as not to be completely connected to the hand-side operation part 20 of the endoscope 12, and to be spaced apart therefrom to some extent.

As illustrated in Fig. 2 and Fig. 3, the hand-side operation part 20 of the endoscope 12 includes a leading end pipe part 36, a central pipe part 42, and the base end pipe part 44. The water supply button 38 and the gas supply button 40 are attached to the leading end pipe part 36, and the angle knob 34 is rotatably attached to the central pipe part 42.

A pipe-like ferrule 50 to which a leading end part of a fluid pipe line 48 is connected is provided on a base end-side end surface 45 of the base end pipe part 44. In addition, the fluid pipe line 48 and the ferrule 50 are rotatably coupled to each other via a rotary joint 51. As illustrated in Fig. 2 or Fig. 3, the ferrule 50 is connected to an ejection hole 52 which is opened on a circumferential surface of the rigid part 28 of the insertion part 22 via a pipe line 54 indicated by a broken line. The pipe line 54 is provided inside of the hand-side operation part 20, and serves to eject the cleaning liquid and the CO₂ gas supplied from the fluid pipe line 48 via the ferrule 50, from the ejection hole 52 to the outside. The fluid pipe line 48 will be described later.

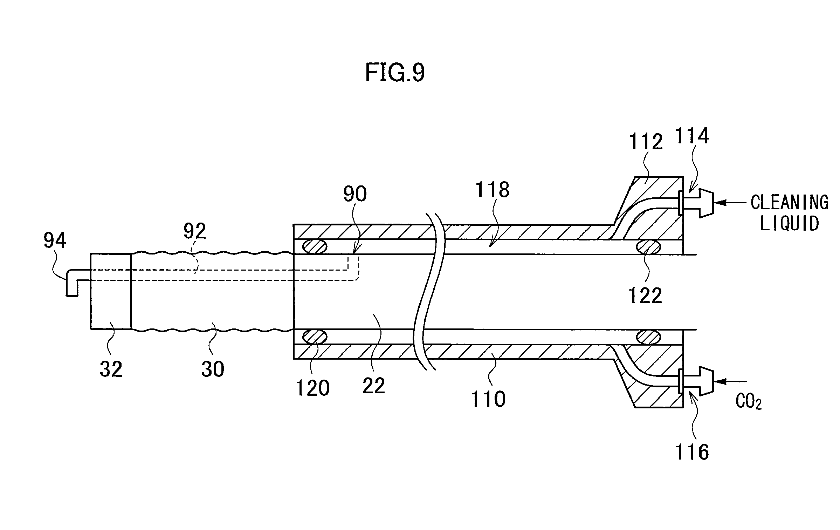

It should be noted that the pipe line which supplies the cleaning liquid and the CO₂ gas is not limited to a pipe line provided inside of the hand-side operation part 20 like the pipe line 54, and hence the cleaning liquid and the CO₂ gas may be supplied via a fluid pipe line connected from the outside (for example, see Fig. 9).

Meanwhile, the universal cord 56 is connected to the base end-side end surface 45 of the base end pipe part 44 via a bend prevention member 58 adjacently to the ferrule 50. The axial direction of the universal cord 56 is set to be the same direction as the axial direction of the ferrule 50, and is also set to be the same direction as the axial direction of the insertion part 22.

In addition, as illustrated in Fig. 2 and Fig. 3, the bending part 30 and the leading end hard part 32 of the insertion part 22 protrude from a leading end opening of the sheath 26 to the outside. Then, as illustrated in Fig. 2, an observation window 66 for taking in subject light and illumination windows 68, 68 for illuminating a subject with illumination light are provided on a leading end surface 35 of the leading end hard part 32 of the insertion part 22.

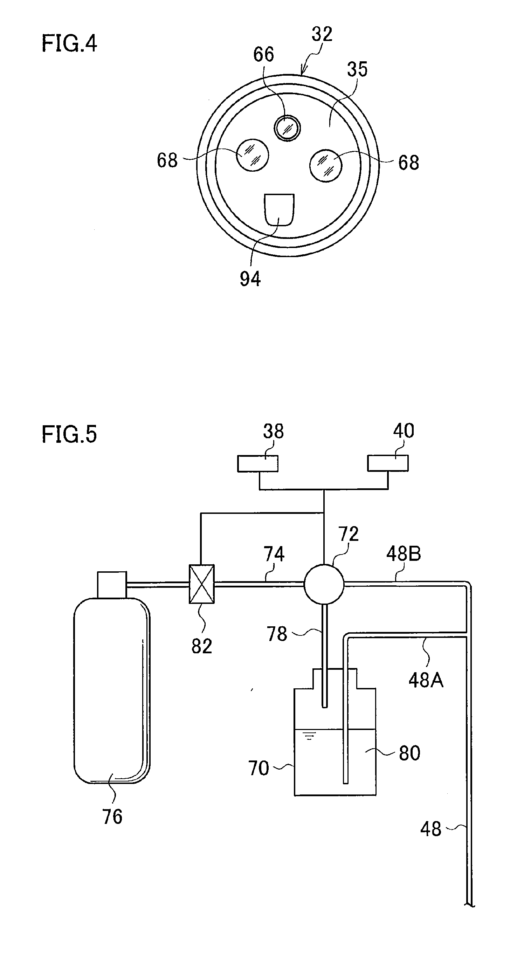

Fig. 4 illustrates the leading end surface 35 of the leading end hard part 32 of the insertion part 22.

As illustrated in Fig. 4, the illumination windows 68, 68 are provided on the leading end surface 35 so as to sandwich the observation window 66. An image pick-up module including an optical system for imaging and a solid state image pick-up element is provided on the inner side of the observation window 66, that is, inside of the leading end hard part 32. A base end part of the signal cable 62 (see Fig. 1) is connected to the image pick-up module. That is, the signal cable 62 is inserted through the universal cord 56, the hand-side operation part 20, and the insertion part 22 to be connected to the image pick-up module.

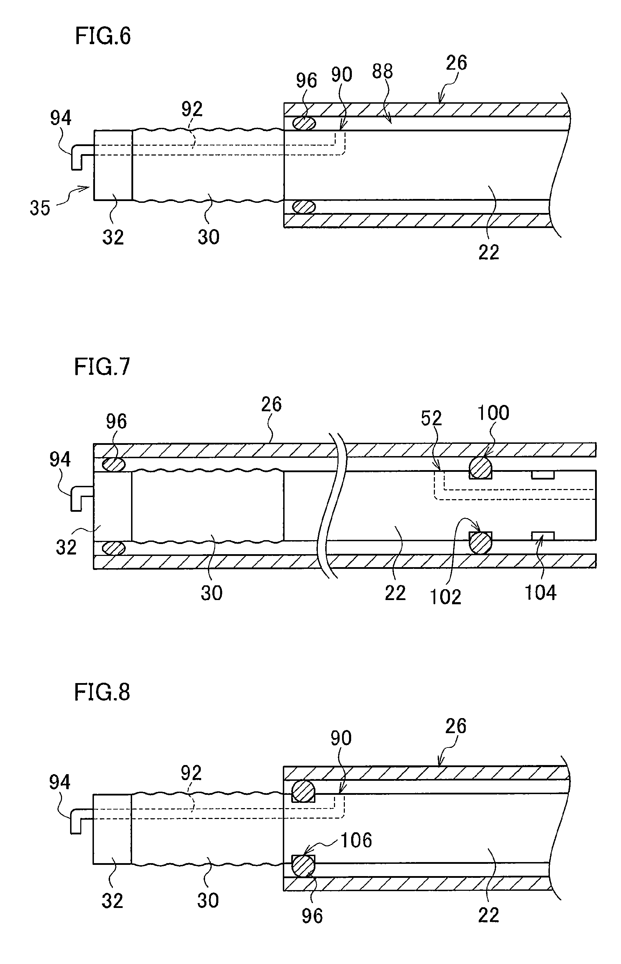

In addition, a light emitting surface of a light guide is placed on the inner side of the illumination windows 68, 68, that is, inside of the leading end hard part 32 so as to be opposed to the illumination windows 68, 68. The light guide is inserted through the insertion part 22, the hand-side operation part 20, and the universal cord 56 to be connected to the LG connector 60. It should be noted that a nozzle 94 (see Fig. 6, the nozzle 94 will be described in detail later) which ejects the cleaning liquid for cleaning the observation window 66 is also formed on the leading end surface 35.

Fig. 5 illustrates the supply system which supplies the cleaning liquid and the CO₂ gas via the fluid pipe line 48.

As described above, the fluid pipe line 48 connected to the ferrule 50 in the base end part of the hand-side operation part 20 is branched into two to become the two pipe lines 48A and 48B.

As illustrated in Fig. 5, the one pipe line 48A is attached to the water supply tank 70, the another pipe line 48B is connected to a gas pipe 74 via a diverter valve 72, and a CO₂ cylinder 76 is connected to the gas pipe 74.

In addition, a pipe line 78 which supplies the CO₂ gas in the CO₂ cylinder 76 to the water supply tank 70 is connected to the diverter valve 72. The water supply tank 70 stores therein a cleaning liquid 80. Here, a physiological saline solution is used as the cleaning liquid 80. In addition, a valve 82 for starting/stopping the supply of the CO₂ gas is attached to the gas pipe 74.

Behaviors of the diverter valve 72 and the valve 82 are controlled by operating the water supply button 38 and the gas supply button 40.

That is, when the water supply button 38 is turned on, the valve 82 is opened, and the diverter valve 72 is operated, so that the pipe line 78 is opened and the pipe line 48B is closed. As a result, the CO₂ gas from the CO₂ cylinder 76 is supplied to the water supply tank 70 via the gas pipe 74 and the pipe line 78, and hence the cleaning liquid 80 in the water supply tank 70 is supplied to the fluid pipe line 48 via the pipe line 48A. Accordingly, the cleaning liquid 80 passes through the pipe line 54 from the ferrule 50 (see Fig. 2) to be ejected from the ejection hole 52 to the outside, then passes through a space between the insertion part 22 and the sheath 26, and flows toward the observation window 66 and the illumination windows 68, 68 illustrated in Fig. 4.

In addition, when the gas supply button 40 of Fig. 5 is turned on, the valve 82 is opened, and the diverter valve 72 is operated, so that the pipe line 78 is closed and the pipe line 48B is opened. As a result, the CO₂ gas from the CO₂ cylinder 76 is supplied to the fluid pipe line 48 via the gas pipe 74 and the pipe line 48B. Accordingly, the CO₂ gas passes through the pipe line 54 from the ferrule 50 (see Fig. 2) to be ejected from the ejection hole 52 to the outside. The CO₂ gas passes through the space between the insertion part 22 and the sheath 26, flows toward the leading end hard part 32 of the insertion part 22, and is blown out toward the observation window 66 and the illumination windows 68, 68 illustrated in Fig. 4. As a result, the observation window 66 and the illumination windows 68, 68 are dried. It should be noted that, when the gas supply button 40 is turned off, the valve 82 is closed. Consequently, the drying operation with the CO₂ gas is stopped.

Fig. 6 is a cross sectional view illustrating the insertion part 22 of the endoscope 12 which is inserted into the sheath 26.

As illustrated in Fig. 6, the bending part 30 and the leading end hard part 32 of the insertion part 22 of the endoscope 12 protrude from the leading end opening of the sheath 26. A hole 90 which is opened in a space 88 between the insertion part 22 and an inner surface of the sheath 26 is formed on a side surface of the insertion part 22 inside of the sheath 26. The hole 90 communicates with a pipe line 92 formed inside of the bending part 30. Then, the pipe line 92 is connected to the nozzle 94 formed on the leading end surface 35 of the leading end hard part 32.

The nozzle 94 serves to eject the cleaning liquid toward the observation window 66 and the illumination windows 68, 68 (see Fig. 2 or Fig. 4) formed on the leading end surface 35 to thereby clean the windows.

In addition, as described above (see Fig. 3), the cleaning liquid is supplied to the space 88 between the insertion part 22 and the inner surface of the sheath 26 via the pipe line 54 provided inside of the hand-side operation part 20 and the ejection hole 52 opened on the circumferential surface of the rigid part 28.

It should be noted that the method of supplying the cleaning liquid to the space 88 is not limited thereto, and, for example, the pipe line 48 may be connected to the connection part 86 of the sheath 26, whereby the cleaning liquid may be supplied directly to the space 88.

The cleaning liquid supplied to the space 88 between the insertion part 22 and the inner surface of the sheath 26 is supplied to the leading end part side with the space 88 serving as a flow channel, and is supplied to the pipe line 92 via the hole 90 formed on the side surface of the insertion part 22. That is, in the present embodiment, the space 88 between the insertion part 22 and the inner surface of the sheath 26 is used as the flow channel of the cleaning liquid.

In this way, because the space 88 between the insertion part 22 and the inner surface of the sheath 26 is used as the flow channel of the cleaning liquid, it is not necessary to provide a pipe line for supplying the cleaning liquid in a thick portion of the sheath 26. Therefore, an inner diameter of the sheath 26 can be made larger, and a diameter of the insertion part 22 to be inserted into the sheath 26 can also be made larger accordingly, so that it becomes possible to add a new function to the insertion part.

In addition, in order to use the space 88 as the flow channel of the cleaning liquid, an O-ring 96 serving as a sealing member for the cleaning liquid is disposed on the inner surface of the sheath 26 on the leading end side from the position of the hole 90 formed on the side surface of the insertion part 22. It should be noted that a similar O-ring is also provided to the connection part 86 of the sheath 26 on the hand-side operation part 20 side, whereby the cleaning liquid can be prevented from leaking from the space 88.

It should be noted that the O-ring 96 serving as the sealing member may be provided on not the inner surface of the sheath 26 but the side surface of the insertion part 22.

In this example, the sheath 26 does not entirely cover the insertion part 22, and is not required to cover a portion of the insertion part 22 on the leading end side from the bending part 30. Therefore, compared with the case where the insertion part 22 is entirely covered, the sheath 26 can be made shorter accordingly, which is advantageous to, for example, perform processing such as sterilization.

However, when the insertion part 22 of the endoscope 12 is inserted into the sheath 26 so that the portion of the insertion part 22 on the leading end side from the bending part 30 protrudes, as describe above, if the insertion part 22 is tried to be inserted into the trocar inserted through the abdominal cavity of a body to be examined, because the bending part 30 which protrudes from the leading end of the sheath 26 is soft, the insertion into the trocar is difficult in some cases.

In view of the above, description is given next on a technique of handling the difficulty when such a member having a leading end formed of a flexible member is inserted into the trocar.

In short, the protrusion of the bending part 30 formed of the flexible member from the leading end opening of the sheath 26 makes the insertion into the trocar difficult, and hence, at the time of the insertion into the trocar, it is advisable to avoid the bending part 30 from protruding from the leading end opening of the sheath 26.

Fig. 7 is a cross sectional view illustrating a state where the insertion part 22 is inserted into the sheath 26 so that the leading end of the insertion part 22 does not protrude from the leading end opening of the sheath 26.

A temporary fixing device (first fixing device) is provided for holding (temporarily fixing) the insertion part 22 of the endoscope 12 at this position inside of the sheath 26. The temporary fixing device is not particularly limited. For example, as illustrated in Fig. 7, the temporary fixing device may include: an O-ring 100 placed on the inner surface of the sheath 26; and a groove 102 which is formed on a circumferential surface of the insertion part 22 so as to engage with the O-ring 100. It should be noted that, in the case of the O-ring 100, as illustrated in Fig. 7, the O-ring 100 and the groove 102 need to engage with each other on the rear side from the ejection hole 52.

Alternatively, instead of causing the O-ring having a seal function to double as a positioning member as described above, for example, a plurality of protrusions (latches) which are dedicated to a positioning function and are formed of an elastic member may be provided along the inner circumferential surface of the sheath 26 and may be engaged with the groove 102. Still alternatively, in addition to the O-ring, such latches dedicated to positioning may be used.

In this way, if the insertion part 22 is inserted into the trocar so that the leading end thereof is not exposed to the outside from the sheath 26, the insertion part 22 can be smoothly inserted into the trocar unlike the case where the leading end thereof is formed of a soft flexible member.

Then, in the state where the insertion part 22 is temporarily fixed as illustrated in Fig. 7, the insertion part 22 inserted into the sheath 26 is inserted into the trocar. After that, the insertion part 22 is further pushed, to thereby cause the portion of the insertion part 22 on the leading end side from the bending part 30 to protrude from the sheath 26 as illustrated in Fig. 6, whereby observation and the like are performed.

It should be noted that, at the time of the observation, the insertion part 22 is fixed (actually fixed) with the portion thereof on the leading end side from the bending part 30 protruding from the sheath 26. An actually fixing device for actual fixing (second fixing device) is not particularly limited. For example, as illustrated in Fig. 7, a groove 104 which can engage with the O-ring 100 may be provided at a predetermined position on the rear side from the groove 102. Alternatively, an engagement member may be provided between the connection part 86 of the sheath 26 and the hand-side operation part 20.

Still alternatively, as illustrated in Fig. 8, a groove 106 which can engage with the O-ring 96 at the leading end of the sheath 26 may be provided on the circumferential surface of the insertion part 22, and the insertion part 22 may be fixed by engaging the O-ring 96 and the groove 106 with each other. It should be noted that, in the case of using the O-ring 96, not only fixing but also sufficient water-tightness needs to be secured.

In the above-mentioned example, the space 88 which is formed when the insertion part 22 of the endoscope 12 is inserted into the sheath 26 is used as the flow channel of the cleaning liquid. Alternatively, the insertion part 22 of the endoscope 12 may be inserted into the trocar instead of the sheath 26, and a space between an inner surface of the trocar and the insertion part 22 may be similarly used as the flow channel of the cleaning liquid.

Fig. 9 is a cross sectional view illustrating an example in which the space which is formed when the insertion part of the endoscope is inserted into the trocar is used as the flow channel of the cleaning liquid.

As illustrated in Fig. 9, the insertion part 22 of the endoscope 12 is inserted into a trocar 110, and the bending part 30 and the leading end hard part 32 protrude to the outside.

A base end part 112 of the trocar 110 is provided with: a ferrule 114 which receives supply of the cleaning liquid; and a ferrule 116 which receives supply of the CO₂ gas, and the cleaning liquid and the CO₂ gas are supplied to a space 118 between the insertion part 22 and an inner surface of the trocar 110 from the ferrules 114 and 116 via pipe lines inside of the base end part 112, respectively.

The insertion part 22 has the same configuration as that in the above-mentioned example, and hence the hole 90 which is opened in the space 118 between the insertion part 22 and the inner surface of the trocar 110 is formed on the side surface of the insertion part 22 inside of the trocar 110. The hole 90 communicates with the pipe line 92 formed inside of the bending part 30. Then, the pipe line 92 is connected to the nozzle 94 formed on the leading end surface 35 of the leading end hard part 32.

In addition, on the inner surface of the trocar 110, an O-ring 120 is placed on the leading end side from the hole 90 of the insertion part 22, and an O-ring 122 is placed also on the base end part 112 side, whereby water-tightness is secured so that the cleaning liquid in the space 118 does not leak to the outside.

In this way, similarly to the sheath 26 described above, the space 118 which is formed when the insertion part 22 of the endoscope 12 is inserted directly into the trocar 110 can be used as the flow channel for supplying the cleaning liquid.

Incidentally, in the case where the space 118 between the trocar 110 and the insertion part 22 is used as the flow channel of the cleaning liquid as described above, a cross sectional area of the flow channel is large, and hence if the endoscope 12 is held at a given position for a long time, the cleaning liquid remaining inside of the flow channel after cleaning of the leading end surface 35 may stay at a given place due to gravity.

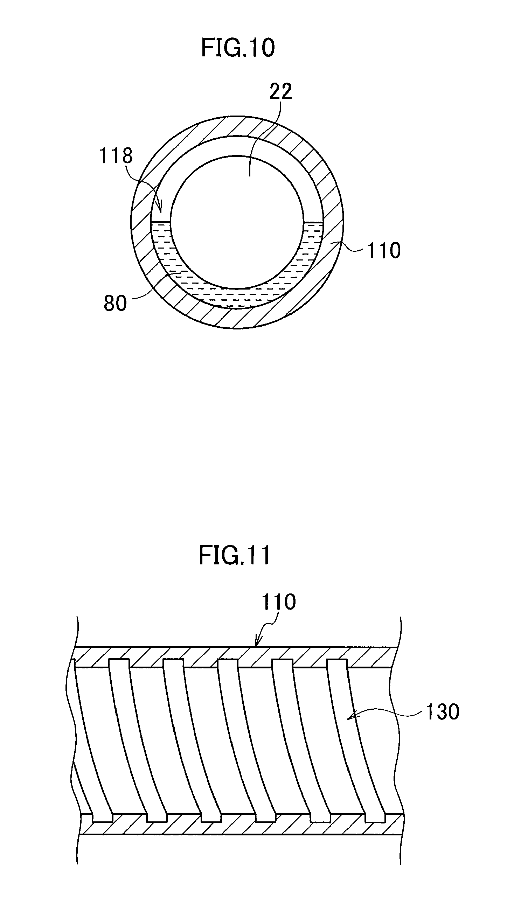

For example, as illustrated in Fig. 10 which is a cross section perpendicular to the longitudinal direction of the trocar 110 into which the insertion part 22 is inserted, the cleaning liquid 80 remaining inside of the space 118 used as the flow channel of the cleaning liquid stays in a lower half portion of the space 118 due to the action of gravity.

If such a cleaning liquid remaining inside of the flow channel (space 118) leaks to the outside for a certain cause, a trouble may occur. Therefore, it is necessary to prevent as far as possible the cleaning liquid from remaining inside of the flow channel, and even if the cleaning liquid should remain, it is necessary to reduce the remaining cleaning liquid to an amount small enough to allow the remaining cleaning liquid to be blown away by supply of the CO₂ gas after the cleaning.

In view of the above, it is desirable to minimize a cross sectional area perpendicular to a flow direction of the flow channel.

For example, as illustrated in Fig. 11 which is a perspective view in which part of an inner wall of the trocar 110 is opened, a single elongated spiral groove 130 which extends over the entire inner surface of the trocar 110 is formed. Then, an outer surface of the insertion part 22 and the inner surface of the trocar 110 are brought as close to each other as possible, to thereby cause the cleaning liquid to flow mainly in the groove 130.

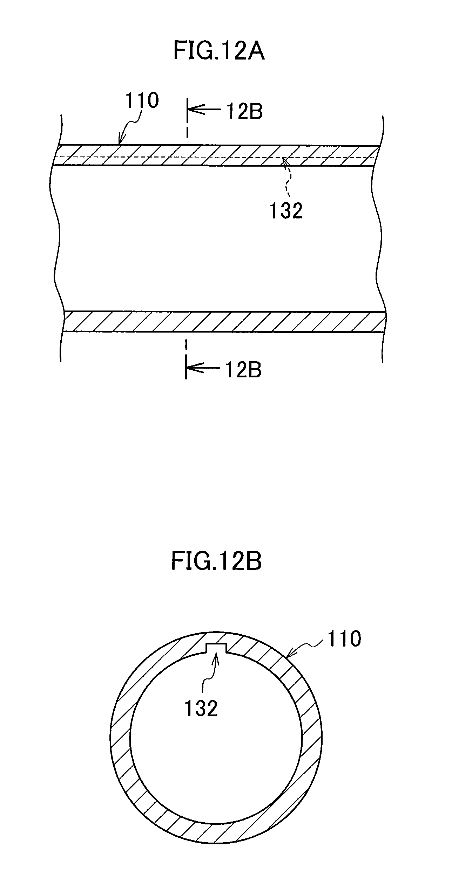

Alternatively, as illustrated in Figs. 12A and 12B, a single narrow groove may be formed in the longitudinal direction of the trocar 110.

That is, Fig. 12A is a cross sectional view in the longitudinal direction of a pipe-like portion of the trocar 110, and Fig. 12B is a cross sectional view which is taken along a broken line 12B-12B in Fig. 12A and is perpendicular to the longitudinal direction. As illustrated in Figs. 12A and 12B, a single narrow groove 132 is formed on the inner surface of the trocar 110 along the longitudinal direction, and the groove 132 may be used as the flow channel of the cleaning liquid.

It should be noted that such grooves may be formed also for the above-mentioned example of the sheath. Similarly in the case of the sheath 26, the inner surface of the sheath 26 and the outer surface of the insertion part 22 are brought closer to each other, to thereby cause the cleaning liquid to flow mainly in such grooves. In this way, a cross sectional area perpendicular to the flow direction of the flow channel is made smaller, whereby the amount of the cleaning liquid remaining inside of the flow channel can be reduced.

Hereinabove, the endoscope, the mantle tube, and the endoscope system according to the present invention have been described in detail. The present invention is not limited to the above-mentioned examples and, as a matter of course, may be variously improved or modified within a range not departing from the scope of the present invention.

## Claims

1. An endoscope system (10), comprising:
a mantle tube (26) into which an insertion part (22) of an endoscope (10) is inserted;
a fluid flow channel which is formed of a space (88) between an outer surface of the endoscope (10) inserted into the mantle tube (26) and an inner surface of the mantle tube (26);
a sealing member (96) which seals the fluid flow channel; and
a pipe line (92) which is formed inside of the insertion part (22) so as to communicate between the fluid flow channel and a nozzle (94) formed on a leading end surface (35) of the insertion part (22), and supply a fluid from the fluid flow channel to the nozzle (94).

2. The endoscope system (10) according to claim 1, wherein the sealing member (96) is provided on the inner surface of the mantle tube (26), and seals a portion between a side surface of the insertion part (22) and the inner surface of the mantle tube (26).

3. The endoscope system (10) according to claim 1, wherein the sealing member is provided on a side surface of the insertion part (22), and seals a portion between the side surface of the insertion part (22) and the inner surface of the mantle tube (26).

4. The endoscope system (10) according to any one of claims 1 to 3, wherein the sealing member (96) is an O-ring which is placed on the inner surface of the mantle tube (26) on a leading end side from a position at which the pipe line (92) formed inside of the insertion part (22) communicates with the fluid flow channel.

5. The endoscope system (10) according to any one of claims 1 to 4, wherein the fluid flow channel is configured by a spiral groove (130) which is formed on the inner surface of the mantle tube (26) continuously from a base end side to a leading end side.

6. The endoscope system (10) according to any one of claims 1 to 4, wherein the fluid flow channel is configured by a linear groove (132) which is formed on the inner surface of the mantle tube (26) continuously in a longitudinal direction from a base end side to a leading end side.

7. The endoscope system (10) according to any one of claims 1 to 6, wherein the mantle tube (26) is one of: a sheath (26) into which the insertion part (22) is inserted; and a trocar (110) which is a support member for inserting the insertion part (22) into a body to be examined.

8. The endoscope system (10) according to any one of claims 1 to 6, further comprising a first fixing device which fixes the insertion part (22) at a position at which a leading end of the insertion part (22) is unexposed to an outside from a leading end of the mantle tube (26).

9. The endoscope system (10) according to claim 8, wherein the first fixing device includes: an O-ring (100) which is placed at a predetermined position on the inner surface of the mantle tube (26); and a groove (102) which is formed at a predetermined position of the insertion part (22) so as to engage with the O-ring (100).

10. The endoscope system (10) according to claim 8, further comprising a second fixing device which fixes the insertion part (22) at a position at which the leading end of the insertion part (22) is exposed to the outside from the leading end of the mantle tube (26).

11. The endoscope system (10) according to claim 10, wherein the second fixing device includes: an O-ring (96) which is placed at a predetermined position on the inner surface of the mantle tube (26); and a groove (106) which is formed at a predetermined position of the insertion part (22) so as to engage with the O-ring (96).

12. The endoscope system (10) according to any one of claims 8 to 11, wherein the mantle tube (26) is a sheath (26) into which the insertion part (22) is inserted.

13. An endoscope (12) comprising an insertion part (22), the insertion part (22) including a side surface on which a sealing member (96) which seals a fluid flow channel is provided, the fluid flow channel being formed of a space (88) between an outer surface of the endoscope (12) and an inner surface of a mantle tube (26) by inserting the insertion part (22) of the endoscope (12) into the mantle tube (26).

14. A mantle tube (26) into which an insertion part (22) of an endoscope(12) is inserted, comprising an inner surface on which a sealing member (96) which seals a fluid flow channel is provided, the fluid flow channel being formed of a space (88) between an outer surface of the endoscope (12) inserted into the mantle tube (26) and the inner surface of the mantle tube (26).
